# EUROPEAN PATENT APPLICATION

(11) **EP 4 607 526 A1**
(43) Date of publication of application: **27.08.2025**
(21) Application number: 23880044.5
(22) Date of filing: 19.09.2023
(51) Int. Cl.: G16H 30/20, G16H 30/40, G16H 20/40, G16H 40/20, G06Q 10/10, H04L 9/00, G06V 10/26, G06V 10/82

(54) **MEDICAL IMAGE RECORD MANAGEMENT METHOD USING BLOCKCHAIN, AND SYSTEM USING SAME**

(30) Priority: 20.10.2022 KR 20220135528
(71) Applicant: Medithinq Co., Ltd., Gyeonggi-do (KR)
(72) Inventor: KIM, Na Young, Yeoju-si Gyeonggi-do 12637 (KR); JUNG, Yoon Sang, Seongnam-si Gyeonggi-do 13385 (KR); SIM, Ji Woo, Yongin-si Gyeonggi-do 16999 (KR)
(74) Representative: Manasse, Uwe
(86) International application number: PCT/KR2023/014126
(87) International publication number: WO 2024/085465

(57) **Abstract**

According to the present invention, in a method for managing medical images using blockchain, there is provided a medical image recording management method comprising: receiving, at a medical image management server, a medical image recorded in relation to surgery or treatment; generating, at the medical image management server, a block on a blockchain network based on data related to the medical image; and updating, through the medical image management server, the block based on new data related to the medical image.

## Description

### Technical Field

The present invention relates to a method for managing records of medical images using blockchain and a system using the same. More specifically, the present invention relates to a method and system for recording a plurality of segmented images generated by dividing medical images through a blockchain network to prevent tampering with medical image records.

### Background Art

Recently, as technology related to multimedia has developed rapidly and is utilized in various fields, technology has been developed in the medical field to produce recordings of surgical scenes for patients so that doctors can present them at medical conferences or use them for other purposes.

In addition, surgical videos are filmed for surgical records and as a defense against potential medical disputes. Endoscopic surgery, using miniature cameras, is common for procedures like joint surgery, allowing visual confirmation of lesions for diagnosis and treatment. Most medical equipment now includes video recording capabilities, leading to a surge in surgical video recording.

These recorded surgical videos have various uses for other doctors, patients, researchers, etc. In particular, if various medical information is added to the surgical video, its utilization and asset value can be increased.

Accordingly, there is a need for a new method and system that can prevent tampering with medical image records and enable secure storage and transaction brokerage. In addition, there is a need for a method and system that can provide medical images more quickly and reliably without interruption during surgery. Furthermore, there is a need for a method and system that can more effectively provide detailed surgical step videos that doctors and medical staff performing surgery can refer to.

### Detailed Description of the Invention

### Technical Problem

The present invention aims to provide a method and system that can, for example, prevent forgery and alteration of surgical videos by separating a recorded video of a surgical scene into a plurality of segmented videos and managing the connection link information and section information of each segmented video with blockchain, and prevent record manipulation or disposal of surgical records.

In addition, the present invention aims to construct a system that can efficiently manage surgical recording videos while reducing the capacity of block data by splitting surgical recording videos into a plurality of segmented videos and constructing blocks using a combination of connection link information and section information.

In addition, the present invention aims to establish a medical image management system that enables transparent management of the use and transaction of recorded surgical videos through transactions via smart contracts and enables monetization through this.

In addition, the present invention aims to provide a method and system that can reduce buffering during video playback that doctors or medical staff performing surgery can refer to by using a cache server and transmit surgical videos more quickly and reliably.

In addition, the present invention aims to provide a cache server system that can provide a plurality of segmented images of a surgical video to a user terminal in a stable streaming manner without delay.

In addition, the present invention aims to provide a video transmission system that can generate and recommend an optimal surgical video based on information related to the surgery being performed.

In addition, the present invention aims to provide a surgical step video transmission system configured to determine more efficient and safe surgical steps based on past surgical record information and to transmit a plurality of video segments generated based on this.

In addition, the present invention aims to provide a method and system that can recommend the optimal detailed surgical steps for a surgical patient using artificial intelligence technology and sequentially provide detailed surgical step videos to medical staff based on this.

The problems to be solved by the present invention are not limited to those described above, and other technical problems not described may be clearly understood by those skilled in the art from the following description.

### Means for Solving the Problem

According to one embodiment of the present invention, in a method for managing medical images using blockchain, the method may include: receiving, at a medical image management server, a medical image recorded in relation to surgery or treatment; generating, at the medical image management server, a block on a blockchain network based on data related to the medical image; and updating, through the medical image management server, the block based on new data related to the medical image.

Herein, the method may further include dividing, at the medical image management server, the medical image into a plurality of segmented images, and sequentially recording the plurality of segmented images in the block based on data related to the plurality of segmented images.

Also, the plurality of segmented images may be sequentially generated based on timestamp information of each section within the medical image.

Also, the plurality of segmented images may further include tag information inserted into the recorded surgical video.

Also, a header of the block may include connection link information, which is information related to a connection link accessible to each of the plurality of segmented images.

Also, the header of the block may further include section information generated based on timestamp information of the start and end points of each of the plurality of segmented images.

Also, the tag information may include at least one of a surgeon, comments related to the surgical situation, a surgical sequence, surgical tools, and surgical events.

Also, the medical image management server may be configured to track accessor information, access IP information, and access time information connected to the connection link as a log record.

Also, the method may further include concluding a transaction contract for the use of the medical image through the medical image management server, and the transaction contract may be configured to settle costs according to preset transaction conditions using a smart contract.

Also, according to another embodiment of the present invention, in a medical image management server for managing medical images using blockchain, the server may include: an image receiving unit configured to receive a medical image recorded in relation to surgery or treatment; a block generation processing unit configured to generate a block on a blockchain network based on data related to the medical image; and a block update processing unit configured to update the block based on new data related to the medical image through the medical image management server.

### Effect of the Invention

According to the present invention, it is possible to provide a method and system that, for example, prevents forgery and alteration of surgical videos by separating a recorded video of a surgical scene into a plurality of segmented videos and managing the connection link information and section information of each segmented video with blockchain, and prevents record manipulation or disposal of surgical records.

Also, according to the present invention, it is possible to construct a system that can efficiently manage surgical recording videos while reducing the capacity of block data by splitting surgical recording videos into a plurality of segmented videos and constructing blocks using a combination of connection link information and section information.

Also, according to the present invention, it is possible to build a medical image management system that enables transparent management of the use and transaction of recorded surgical videos through transactions via smart contracts and enables monetization through this.

Also, according to the present invention, it is possible to provide a method and system that can reduce buffering during video playback that doctors or medical staff can refer to by using a cache server, and transmit surgical videos more quickly and reliably.

Also, according to the present invention, it is possible to provide a cache server system that can provide a plurality of segmented images of a surgical video to a user terminal in a stable streaming manner without delay.

Also, according to the present invention, it is possible to provide a video transmission system capable of generating and recommending an optimal surgical video based on information related to the ongoing surgery.

Also, according to the present invention, it is possible to provide a surgical stage video transmission system configured to determine more efficient and safer detailed surgical stages of surgery based on past surgical record information and to transmit a plurality of video segments generated based on this.

In addition, according to the present invention, it is possible to provide a method and system that can recommend optimal detailed surgical steps suitable for a surgical patient using artificial intelligence technology, and sequentially provide detailed surgical step images based on this to medical staff.

The effects of the present invention are not limited to those mentioned above, and other effects not mentioned may be clearly understood by those skilled in the art from the following description.

### Brief Description of Drawings

FIG. 1 is a conceptual diagram illustrating the configuration of a system for managing records of medical images using blockchain according to an embodiment of the present invention.
FIG. 2 is a block diagram for explaining the configuration of a medical image management server according to an embodiment of the present invention.
FIG. 3 is an exemplary diagram illustrating the generation of blocks on a blockchain network for a plurality of segmented images related to a medical image according to an embodiment of the present invention.
FIG. 4 is an exemplary diagram for explaining the header structure of a block according to an embodiment of the present invention.
FIG. 5 is a flowchart for explaining a method for managing medical images using blockchain according to an embodiment of the present invention.
FIG. 6 is a conceptual diagram for explaining the configuration of a surgical video transmission system using a cache server according to an embodiment of the present invention.
FIG. 7 is a block diagram for explaining the configuration of a cache server according to an embodiment of the present invention.
FIG. 8 is an exemplary diagram for explaining a method for providing a plurality of video segments related to a surgical video using a cache server according to an embodiment of the present invention.
FIG. 9 is a flowchart for explaining a surgical video transmission method using a cache server according to an embodiment of the present invention.
FIG. 10 is a conceptual diagram for explaining the configuration of a surgical step video transmission system using a cache server according to an embodiment of the present invention.
FIG. 11 is an exemplary diagram for explaining a configuration for recommending detailed surgical steps for a surgical patient according to an embodiment of the present invention.
FIG. 12 is an exemplary diagram for explaining a method for providing video of detailed surgical steps using a cache server according to an embodiment of the present invention.

### Mode for Implementing of the Invention

Hereinafter, the present invention will be described in detail with reference to the accompanying drawings so that those skilled in the art to which the present invention pertains can easily carry it out. However, the present invention may be embodied in many different forms and should not be construed as limited to the embodiments set forth herein.

The terminology used herein is for the purpose of describing particular embodiments and is not intended to limit the invention. As used herein, the singular forms are intended to include the plural forms as well, unless the context clearly indicates otherwise.

The terms "comprises" and "comprising," in this specification, do not exclude the presence or addition of one or more other 'elements, steps, operations and/or units'.

Also, terms including ordinal numbers such as first and second used in the present invention may be used to describe components, but the components should not be limited by the terms. Such terms are only used for the purpose of distinguishing one component from another. In addition, in describing the present invention, if it is determined that a detailed description of related known technology may obscure the gist of the present invention, the detailed description thereof will be omitted.

In addition, the components shown in the embodiments of the present invention are independently illustrated to represent different characteristic functions, and it does not mean that each component is made of separate hardware or one software component unit. That is, each component is described by listing each component for convenience of explanation, and at least two of the components may be combined to form one component, or one component may be divided into a plurality of components to perform a function. Embodiments in which each component is integrated and embodiments in which each component is separated are also included in the scope of the present invention unless they depart from the essence of the present invention.

Hereinafter, embodiments according to the present invention will be described in detail with reference to the accompanying drawings. The configuration of the present invention and the operational effects thereof will be clearly understood through the following detailed description.

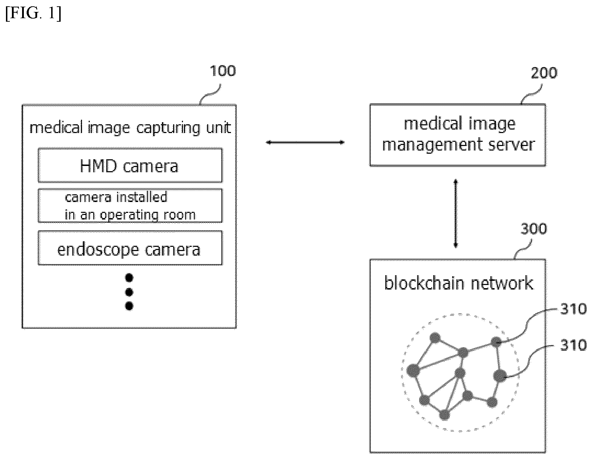

FIG. 1 is a conceptual diagram illustrating the configuration of a system for managing records of medical images using blockchain according to an embodiment of the present invention.

The medical image management system may be composed of a medical image capturing unit (100), a medical image management server (200), and a blockchain network (300). The medical image capturing unit (100) records images related to surgery, treatment, medical examination, etc. in spaces such as operating rooms, treatment rooms, or examination rooms in a hospital, and is configured to be able to transmit the recorded medical images to the medical image management server (200). The medical image capturing unit (100) may take the form of a plurality of cameras or CCTVs of various types, such as, for example, a head-mounted display (HMD) camera that can be worn by medical staff such as doctors, a camera installed in an operating room, or an endoscope camera used in endoscopic surgery. For example, a camera installed in an operating room may be installed on the ceiling to specify the coordinates of each object in the initial real-time image to identify the initial position, and to track the movement by grasping the surgical scene and the movements of the surgeon. In addition, by using image recognition technology, all objects such as surgical tools to be photographed can be tracked for movement, so that the surgical scene can be automatically photographed and recorded.

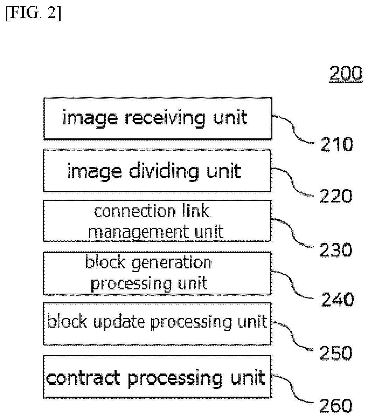

The medical image management server (200) is configured to receive medical images recorded in relation to surgery or treatment using the medical image capturing unit (100), generate a block on the blockchain network (300) based on data related to the medical images, and update the block based on new data related to the medical images. The medical image management server (200) may include a memory for storing data and instructions, and a processor for executing the instructions, to manage information related to each module configured to receive and manage medical images. A more specific configuration of the medical image management server (200) will be described with reference to FIG. 2.

The medical image management server (200) may be a server corresponding to any one of the blockchain nodes constituting the blockchain network (300), or a server for managing the blockchain network (300). Blockchain technology is based on a peer-to-peer (P2P) method, and stores data to be managed in a distributed data storage environment called a block, which is formed by small data linked in a chain form, so that no one can arbitrarily modify it and anyone can view the results of the change. A block records all transaction details or status information propagated to users before the block was discovered, and this is transmitted to all users in the same way using the P2P method, so the transaction details or status information cannot be arbitrarily modified or omitted. In the present invention, data or a bundle of data for managing medical images stored and updated through the blockchain network (300) is referred to as a 'block'.

FIG. 2 is a block diagram for explaining the configuration of a medical image management server according to an embodiment of the present invention.

The medical image management server (200) may include an image receiving unit (210), an image dividing unit (220), a connection link management unit (230), a block generation processing unit (240), a block update processing unit (250), and a contract processing unit (260), etc., and these components may include a program or program module that can be executed by one or more processors. The programs or program modules included in the medical image management server (200) may be configured in the form of an operating system, an application program, or a program, and may be physically stored on various types of storage devices that are widely used. Such programs or program modules may include various forms for performing one or more routines, subroutines, programs, objects, components, instructions, data structures, and specific tasks or executing specific data types, and are not limited to these forms.

First, the image receiving unit (210) is configured to receive medical images recorded in relation to surgery or treatment using the medical image capturing unit (100), and may be connected to the medical image capturing unit (100) using wired or wireless Internet.

The image dividing unit (220) is configured to divide the medical image into a plurality of segmented images, and based on data related to the divided plurality of segmented images, blocks corresponding to each segmented image information can be generated and sequentially recorded through the block generation processing unit (240). For example, the image dividing unit (220) may sequentially generate a plurality of segmented images from one medical image based on timestamp information set for each section in the medical image. For example, the timestamp information may be set for each detailed procedure in the surgery by the surgeon or medical staff who performed the surgery, or may be automatically set using artificial intelligence technology. In addition, the plurality of segmented images may further include tag information inserted into the recorded surgical video, and the tag information may include at least one of a surgeon, comments related to the surgical situation, a surgical sequence, surgical tools, and surgical events. Such tag information may be input by medical staff in association with each timestamp information, or related information may be automatically input using artificial intelligence technology.

The block generation processing unit (240) may be configured to generate a block on the blockchain network (300) based on data related to the medical image. For example, it may be configured to generate a new block for each of the plurality of segmented images. Also, the header of the generated block may include information related to a connection link accessible to each of the plurality of segmented images. In addition, the header of the generated block may further include section information generated based on timestamp information of the start and end points of each of the plurality of segmented images.

The block update processing unit (250) may be configured to update block information based on tag information, etc. for the generated block. For example, it may include additional tag information corresponding to each segmented image, browsing history, transaction history, usage history, etc. For example, accessor information, access IP information, and access time information connected through the connection link of the segmented image can be tracked as a log record, and such access information can be recorded and updated in the body of the block corresponding to the segmented image.

The contract processing unit (260) is configured to conclude various transaction contracts for the use of medical images and process related processes, and such transaction contracts may be configured to settle costs according to preset transaction conditions using a smart contract. The contract processing unit (260) may be configured to generate a block for a smart contract on the blockchain network (300) based on data related to the use of the medical image through a smart contract, and may include information on the usage period of the medical image, that is, information on the start date and end date, information on access and editing rights of the image, etc.

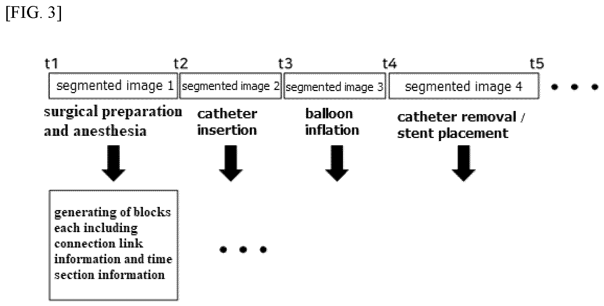

FIG. 3 is an exemplary diagram illustrating the generation of blocks on a blockchain network for a plurality of segmented images related to a medical image according to an embodiment of the present invention.

Referring to FIG. 3, for example, a medical image related to a stent procedure or surgery may be divided into four segmented images according to each step, that is, 1) surgical preparation and anesthesia, 2) catheter insertion, 3) balloon inflation, and 4) catheter removal and stent placement, and each segmented image may include section information related to the start and end points of t1~t2, t2~t3, t3~t4, and t4~t5 as timestamp information. Different accessible connection links may be assigned to each divided segmented image, and connection link information, which is information related to the connection link corresponding to each segmented image, may be generated. In this way, a plurality of blocks each including connection link information and time section information can be generated for each of the plurality of segmented images into which the medical image is sequentially divided.

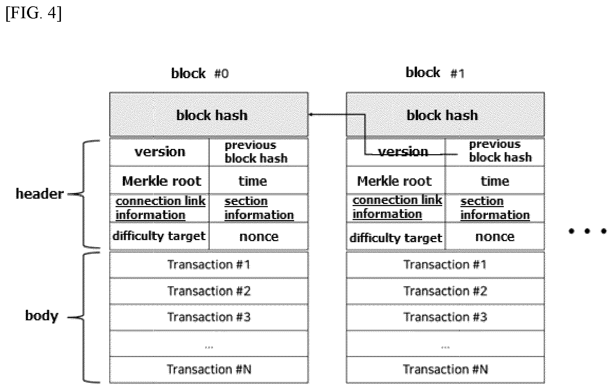

FIG. 4 is an exemplary diagram for explaining the header structure of a block according to an embodiment of the present invention.

The structure of a block may be largely composed of a block hash that serves as an identifier of the block, a header that has basic information, and a body that has transaction information. Here, the header generally includes information such as version, previous block hash, Merkle root, time, difficulty target, and nonce, and the block according to the present invention may further include, in the header, connection link information related to a connection link accessible to each of the plurality of segmented images and section information generated based on timestamp information of the start and end points of each of the plurality of segmented images.

Through such a block structure, it is possible to prevent tampering with medical image records by matching link information accessible to surgical image segments or still cuts for each segmented and distributed ledger and recording them using blockchain. For example, the link information can be encrypted, and the storage space where the image or still cut is stored can be set to read-only so that the image at the access location cannot be changed.

The present invention can provide a system that can efficiently manage surgical recording videos while reducing the capacity of block data by splitting surgical recording videos into a plurality of segmented videos and constructing blocks using a combination of connection link information and section information, and can solve the problem of surgical history being discarded due to expiration of the storage period or major content related to the surgery being altered.

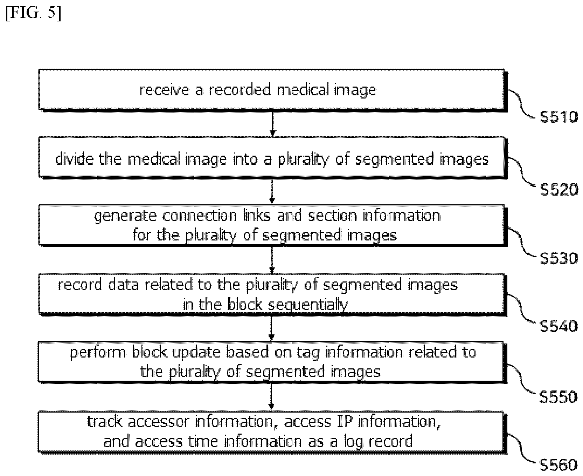

FIG. 5 is a flowchart for explaining a method for managing medical images using blockchain according to an embodiment of the present invention.

Referring to FIG. 5, first, a medical image captured and recorded through the medical image capturing unit (100) may be received through the image receiving unit (210) of the medical image management server (200) (S510).

The medical image management server (200) may sequentially divide the medical image into a plurality of segmented images through the image dividing unit (220) (S520).

The medical image management server (200) may generate connection links for the plurality of segmented images through the connection link management unit (230) and generate section information for each segmented image (S530).

The medical image management server (200) may sequentially record data related to the plurality of segmented images in the block through the block generation processing unit (240) (S540). At this time, the header of the block may include connection link information related to a connection link accessible to each of the plurality of segmented images, and section information generated based on timestamp information of the start and end points of each of the plurality of segmented images.

In addition, the medical image management server (200) may perform block update based on tag information related to the plurality of segmented images through the block update processing unit (250) (S550). For example, the block may be updated based on additional tag information corresponding to each segmented image, browsing history, transaction history, usage history, etc.

In addition, the medical image management server (200) may track accessor information, access IP information, and access time information as a log record (S560). By tracking the access-related information connected through the link, access information can be monitored. In addition, the block can be updated based on the access-related information to prevent tampering with the access record.

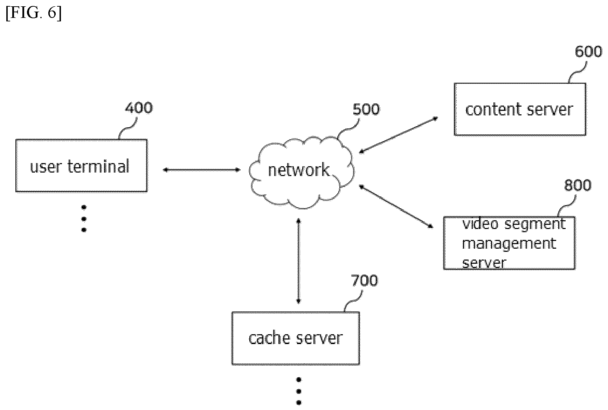

FIG. 6 is a conceptual diagram for explaining the configuration of a surgical video transmission system using a cache server according to an embodiment of the present invention.

Referring to FIG. 6, the user terminal (400) is a device configured to be used by a user such as a doctor or medical staff performing surgery, treatment, medical examination, etc., and to be able to access, for example, a content server (600), a cache server (700), and a video segment management server (800). Specifically, it may be any one of a smartphone, a tablet computer, a desktop computer, a laptop computer, a notebook, a workstation, a Personal Digital Assistant (PDA), a portable computer, a wireless phone, a mobile phone, an e-book, a portable multimedia player (PMP), a portable game console, a digital camera, a television, a wearable device, a head-mounted display (HMD), or an AI (artificial intelligence) speaker, but is not limited thereto. In addition, the user terminal (400) may include a display unit for providing a medical image screen or may be connected to the display unit and configured to be controllable.

The user terminal (400) is configured to be able to communicate with various servers, such as the content server (600), the cache server (700), and the video segment management server (800), through the network (500). The network (500) is a component for performing wired and wireless communication for data transmission and reception between the user terminal (400) and the plurality of servers (600, 700, 8000). If the network is a wireless communication network, it may include cellular communication or short-range communication. For example, cellular communication may include at least one of Long-Term Evolution (LTE), LTE Advanced (LTE-A), 5th Generation (5G), Code Division Multiple Access (CDMA), Wideband CDMA (WCDMA), Universal Mobile Telecommunications System (UMTS), Wireless Broadband (WiBro), or Global System for Mobile Communications (GSM). In addition, short-range communication may include at least one of Wireless Fidelity (Wi-Fi), Bluetooth, Zigbee, Near Field Communication (NFC), or Radio Frequency Identification (RFID). However, the communication method is not limited thereto, and technologies of wireless communication to be developed in the future will also be included.

Next, the content server (600) may be configured to store a plurality of surgical videos, and may be configured to store videos recorded in relation to surgery, treatment, medical examination, etc. in spaces such as operating rooms, treatment rooms, or examination rooms in a hospital through various types of cameras through the medical image capturing unit (100).

The cache server (700) is configured to receive and store at least a part of a surgical video related to the surgery among a plurality of surgical videos stored from the content server (600), and to transmit at least a part of the surgical video to the user terminal (400) when there is a request for a reference video from the user terminal (400). In addition, the cache server (700) may be selected as the most advantageous cache server based on the proximity to the location of the user terminal (400) or the response speed among a plurality of cache servers.

The video segment management server (800) is configured to receive surgical-related information including the date and time of surgery and the type of surgery, determine a surgical video related to the surgery among a plurality of surgical videos stored in the content server (600) based on the surgical-related information, and divide the related surgical video into a plurality of video segments having a predetermined capacity or less.

The divided plurality of video segments are sequentially generated based on timestamp information of each section within the surgical video, and the video segment management server (800) may be configured to deliver at least one video segment corresponding to the front part of the plurality of video segments to the cache server (700).

**In** addition, the video segment management server (800) may be configured to deliver at least one video segment to the cache server (700) before the surgical date and time based on a predetermined surgical date and time. For example, based on a predetermined criterion, the video segment may be delivered to the cache server (700) in advance, for example, one day or one hour before the surgical date and time, and set to be stored in the cache server (700).

**In** addition, the video segment management server (800) may be configured to determine a related surgical video among a plurality of surgical videos stored in the content server (600) based on a similarity judgment with reference video information previously used in the user terminal (400). That is, the surgical video that is likely to be referred to during the surgery can be determined by using past history data that the user of the user terminal (400) has previously referred to or searched for.

In addition, when at least a predetermined proportion of at least one video segment is streamed on the user terminal (400), it is determined that the user is referring to the video segment, and the cache server (700) requests the video segment management server (800) for the remaining video segments excluding the front part of the video segment, receives and stores them. By storing the remaining video segments in the cache server (700) in advance, transmission delay can be minimized when streaming the surgical video through the user terminal (400).

In addition, when all of the plurality of video segments are streamed on the user terminal (400), the cache server (700) may be configured to transmit at least a part of a second surgical video corresponding to the next turn of the transmitted surgical video to the user terminal (400). In addition, when at least a predetermined proportion of at least one video segment or the entire video segment of the second surgical video is streamed on the user terminal (400), it is determined that the user is referring to the video segment of the second surgical video, and the cache server (700) requests the video segment management server (800) for the remaining video segments excluding the above front part, receives and stores them.

In addition, the video segment management server (800) may determine at least one major video segment among the plurality of video segments based on at least one of the movement of surgical tools, the amount of blood generated, and the degree of change of organs related to the surgery through video analysis of the plurality of video segments using artificial intelligence technology, and may be configured to deliver the major video segment among the plurality of video segments to the cache server (700). In this way, the video segment management server (800) can efficiently utilize the limited storage space of the cache server (700) by determining a major video segment that is considered important among a plurality of video segments and delivering only the major video segment to the cache server (700).

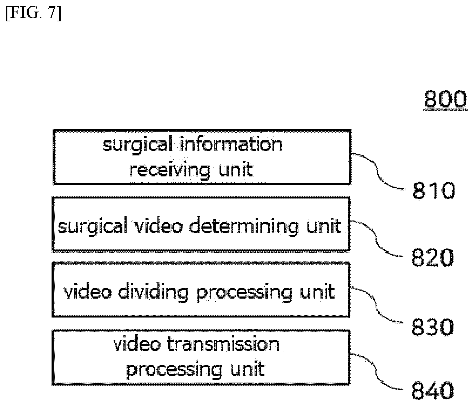

FIG. 7 is a block diagram for explaining the configuration of a cache server according to an embodiment of the present invention.

Referring to FIG. 7, the video segment management server (800) according to the present invention may include a surgical information receiving unit (810), a surgical video determining unit (820), a video dividing processing unit (830), and a video transmission processing unit (840), and these components may include a program or program module that can be executed by one or more processors. The programs or program modules included in the video segment management server (800) may be configured in the form of an operating system, an application program, or a program, and may be physically stored on various types of storage devices that are widely used. Such programs or program modules may include various forms for performing one or more routines, subroutines, programs, objects, components, instructions, data structures, and specific tasks or executing specific data types, and are not limited to these forms.

First, the surgical information receiving unit (810) may be configured to receive surgical-related information including the date and time of surgery, the subject of surgery, the location of the surgical site, and the type of surgery from the user terminal (400) or other servers or terminals that manage surgical information.

The surgical video determining unit (820) is configured to determine a surgical video to be provided as a surgical reference video to the user terminal (400), and may be configured to determine a related surgical video among a plurality of surgical videos stored in the content server (600) based on the received surgical-related information. In addition, the surgical video determining unit (820) may determine a related surgical video among a plurality of surgical videos stored in the content server (600) based on a similarity judgment with reference video information previously used in relation to the type of surgery in the user terminal (400) by using previous history information of the user terminal (400).

In addition, the surgical video determining unit (820) may determine at least one major video segment among the plurality of video segments based on at least one of the movement of surgical tools, the amount of blood generated, and the degree of change of organs related to the surgery by using video analysis of the plurality of video segments through artificial intelligence technology or worker processing, and may be configured to deliver only the major video segment among the plurality of video segments to the cache server (700). In this way, it is possible to efficiently utilize the limited storage space of the cache server (700) by determining a major video segment that is considered important among a plurality of video segments and delivering only the major video segment to the cache server (700), and from the perspective of medical staff, it is possible to efficiently utilize time by referring only to major video segments in a limited time.

The video dividing processing unit (830) may be configured to divide the related surgical video determined by the surgical video determining unit (820) into a plurality of video segments having a predetermined capacity or less. At this time, the plurality of video segments are sequentially generated based on timestamp information of each section within the surgical video, and the video segment management server (800) may be configured to deliver at least one video segment corresponding to the front part of the sequentially generated plurality of video segments to the cache server (700) through the video transmission processing unit (840).

The video transmission processing unit (840) is configured to perform transmission processing and transmission schedule management of the video to be delivered to the cache server (700), and may determine one cache server connected to the user terminal (400) based on proximity to the location of the user terminal (400) or the response speed of each cache server. In addition, the video transmission processing unit (840) may be configured to deliver at least one video segment to the cache server (700) before the surgical date and time based on the surgical date and time information included in the surgical-related information.

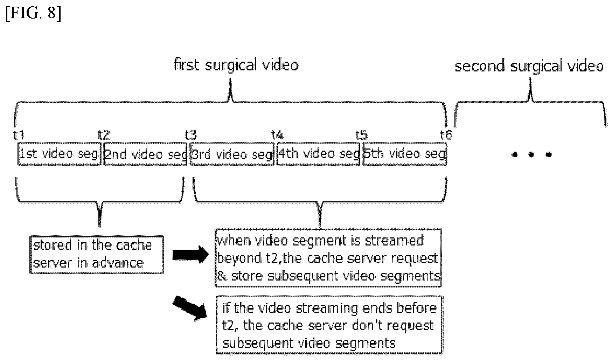

FIG. 8 is an exemplary diagram for explaining a method for providing a plurality of video segments related to a surgical video using a cache server according to an embodiment of the present invention.

For example, the scheduled surgery may include a plan to sequentially perform two types of surgery, a first surgery related to a first surgical video and a second surgery related to a second surgical video.

First, the first surgical video determined as a reference video in relation to the first surgery may be one in which five video segments are sequentially generated based on timestamp information (t1~t6) of each section, and the first video segment and the second video segment corresponding to the front part may be stored in the cache server in advance. At this time, when all of the first video segment is streamed and viewed beyond the time point of t2 through the user terminal (400), for example, the cache server (700) may be configured to request subsequent video segments, for example, the third video segment, the fourth video segment, and the fifth video segment, from the video segment management server (800), receive them, and store them. By delivering and storing subsequent video segments in advance in the cache server (700) according to whether or not the divided video segments are viewed, it is possible to ensure that the video can be viewed without delay for the surgical video to be viewed by the user.

Meanwhile, if the video streaming ends before the predetermined criterion of t2 through the user terminal (400), it is determined that the reference video is not needed, and the cache server (700) may be configured not to request subsequent video segments, for example, video segments after the third video segment. In addition, in order to efficiently manage the storage capacity in the cache server (700), if the video streaming is terminated before a certain point in time, the video segments may be determined to be of no interest to the user and may be configured to be deleted.

In addition, when the streaming viewing of the first surgical video is completed through the user terminal (400), the cache server (700) may be configured to transmit a video segment at the front part as at least a part of the second surgical video corresponding to the next turn to the user terminal (400). At this time, if the video segment at the front part of the second surgical video is not stored in the cache server (700), for example, at the time point of t4 or t5 before the viewing of the first surgical video is completed, the cache server (700) may request the video segment at the front part of the second surgical video from the video segment management server (800), receive it, and store it in advance.

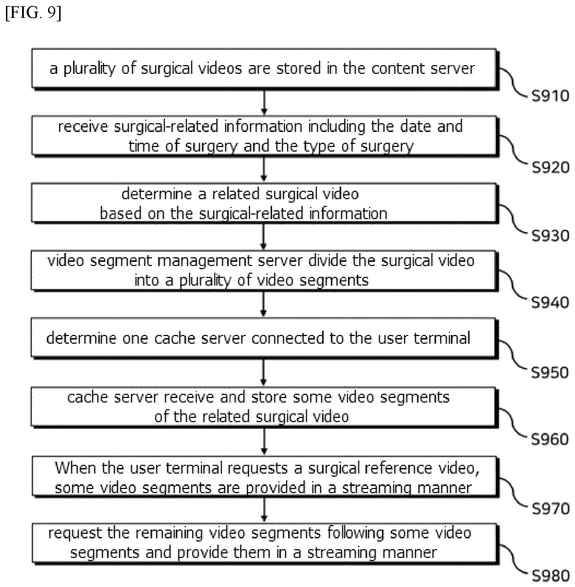

FIG. 9 is a flowchart for explaining a surgical video transmission method using a cache server according to an embodiment of the present invention.

First, a plurality of surgical videos may be stored in the content server (600) (S910). At this time, the surgical video may include the type of surgery, the subject of surgery, timestamp information for each section, and tag information.

The video segment management server (800) may receive surgical-related information including the date and time of surgery and the type of surgery through the surgical information receiving unit (810) (S920).

The video segment management server (800) may determine a related surgical video based on the surgical-related information through the surgical video determining unit (820) (S930).

The video segment management server (800) may divide the surgical video into a plurality of video segments through the video dividing processing unit (830) (S940). For example, the surgical video may be one in which a plurality of video segments are sequentially generated based on timestamp information of each section.

The video segment management server (800) may determine one cache server connected to the user terminal (400) based on proximity to the location of the user terminal (400) or the response speed of each cache server through the video transmission processing unit (840) (S950).

The determined cache server (700) may receive and store some video segments of the related surgical video from the video segment management server (800) (S960).

When the user terminal (400) requests a surgical reference video, some video segments may be provided in a streaming manner through the determined cache server (700) (S970).

In addition, the user terminal (400) may request the remaining video segments following some video segments and provide them in a streaming manner through the cache server (700) (S980). When the user terminal (400) streams a certain percentage or more of a plurality of segments located at the front of the surgical video, the cache server (700) requests the remaining video segments from the video segment management server (800), receives them, and stores them so that the entire video can be viewed without delay.

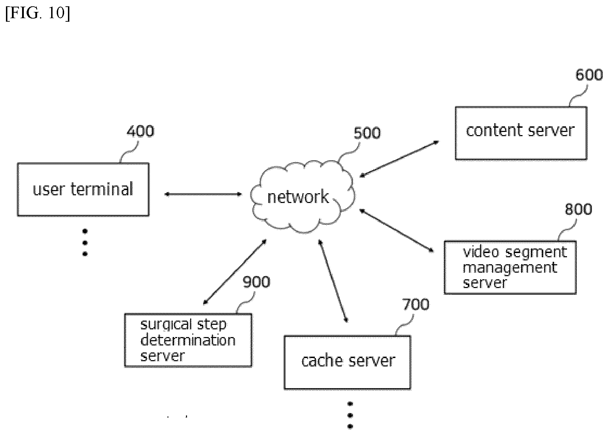

FIG. 10 is a conceptual diagram for explaining the configuration of a surgical step video transmission system using a cache server according to an embodiment of the present invention.

The surgical step video transmission system of FIG. 10 is a form in which a surgical step determination server (900) is added to the configuration of the surgical video transmission system using a cache server shown in FIG. 6.

Here, the content server (600) may be configured to store a plurality of surgical videos.

The video segment management server (800) may receive surgical-related information including the type of surgery, and may be further configured to divide and store a plurality of surgical videos stored in the content server (600) based on detailed surgical steps based on the received surgical-related information.

The surgical step determination server (900) may be configured to determine detailed surgical steps based on the type of surgery of the surgical patient and to determine each video segment related to each detailed surgical step.

**In** addition, the surgical step determination server (900) may be configured to determine detailed surgical steps based on record information of a plurality of previous surgical patients who underwent the same type of surgery as the surgical patient. In addition, the record information of the previous surgical patient may include at least one of gender, age, time of surgery, underlying disease, history of other surgeries, prognosis after surgery, and recovery period after surgery. That is, when determining detailed surgical steps, it is possible to utilize surgical steps and conditions that have a good prognosis and recovery period under conditions similar to those of the current patient by using the record information of previous surgical patients.

In addition, the surgical step determination server (900) may be configured to determine detailed surgical steps by combining detailed surgical steps of a plurality of previous surgical patients. For example, it may be configured to determine the detailed surgical steps of the current surgical patient by combining the detailed surgical steps of a first previous surgical patient who underwent the same type of surgery as the current surgical patient and the different detailed surgical steps of a second previous surgical patient who underwent the same type of surgery as the current surgical patient.

In addition, the surgical step determination server (900) can derive detailed surgical steps that have the best prognosis and recovery period under similar conditions of the current patient by using the record information of previous patients using a learning model using artificial intelligence, and accordingly, it can be configured to recommend the optimal detailed surgical steps to the current surgical patient through the artificial intelligence learning model.

The cache server (700) receives and stores each of a plurality of video segments related to each detailed surgical step from the video segment management server (800), and transmits a plurality of video segments related to the detailed surgical steps determined by the surgical step determination server (900) to the user terminal (400) when there is a request for a detailed surgical step video from the user terminal (400). At this time, the cache server (700) may be determined as the cache server at the optimal location based on the proximity to the location of the user terminal (400) or the response speed among a plurality of cache servers.

In addition, the cache server (700) checks whether each video segment related to each detailed surgical step determined by the surgical step determination server (900) is stored, and for video segments not stored in the cache server (700), it may be configured to receive and store the video segment from the video segment management server (800) based on the surgical schedule of the surgical patient. For example, the cache server (700) may check whether each video segment related to each detailed surgical step is stored before the surgical date and time of the surgical patient, for example, one hour or one day before, and request the video segment management server (800) for video segments that are not stored, and store the video segments in advance before the surgical date and time.

The user terminal (400) may be configured to receive a plurality of video segments based on the detailed surgical steps determined by the surgical step determination server (900).

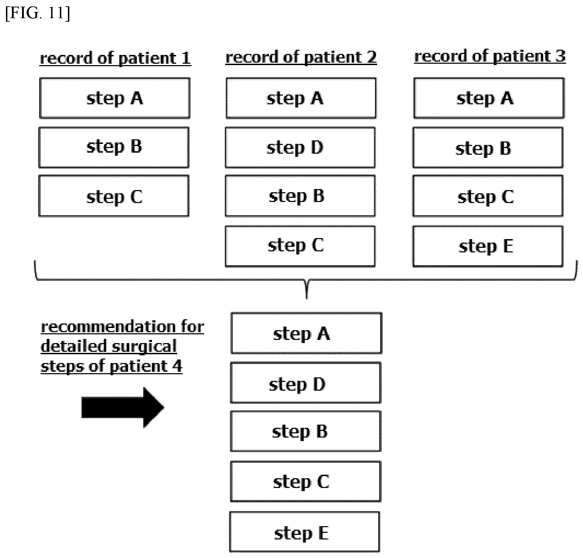

FIG. 11 is an exemplary diagram for explaining a configuration for recommending detailed surgical steps for a surgical patient according to an embodiment of the present invention.

One surgery can be divided into several detailed surgical steps, and video segments can be divided for each detailed surgical step. In FIG. 11, it is assumed that patient 1, patient 2, and patient 3 have records of having undergone the same type of surgery in the past, and patient 4 is scheduled to undergo the same type of surgery.

For example, looking at the previous surgical record of patient 1, it has detailed surgical steps consisting of three steps: step A, step B, and step C. The previous surgical record of patient 2 has a total of four detailed surgical steps with step D added between step A and step B. In addition, in the previous surgical record of patient 3, step E is added after step A, step B, and step C, resulting in a total of four detailed surgical steps. As such, even in the case of the same type of surgery, some detailed surgical steps may be added or changed depending on the patient's condition and the situation of the surgery, and as such surgical data is accumulated, probabilistic judgment on the results of detailed surgical steps becomes possible.

The detailed surgical steps determined by the surgical step determination server (900) for patient 4, who is scheduled to undergo the same type of surgery as patients 1 to 3, are a combination of the detailed surgical steps of patients 1 to 3, and include a total of five detailed surgical steps consisting of step A, step D, step B, step C, and step E, and detailed surgical step images corresponding to these five steps may be provided to the medical staff as reference images. The recommendation proposal and determination of such detailed surgical steps can derive detailed surgical steps that have the best prognosis and recovery period under similar conditions of the current patient by using the record information of previous patients using, for example, a learning model using artificial intelligence, and can find and recommend detailed surgical step conditions that have the best prognosis and recovery conditions under patient environment conditions such as the same gender, similar age, and similar underlying diseases.

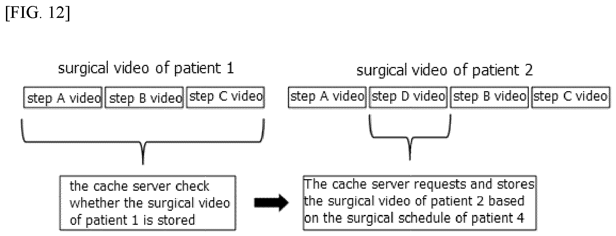

FIG. 12 is an exemplary diagram for explaining a method for providing video of detailed surgical steps using a cache server according to an embodiment of the present invention.

Referring to FIG. 12, the detailed surgical steps of patient 4 determined by the surgical step determination server (900) may include the surgical video of patient 1 as step A video, step B video, and step C video, and may also include step D video of the surgical video of patient 2.

At this time, the cache server (700) may first check whether the surgical video of patient 1 and the video of step D of the surgical video of patient 2 are stored before the surgical date and time of the surgical patient. For example, as shown in FIG. 12, if it is determined that all of the surgical video of patient 1, that is, step A video, step B video, and step C video, are stored, but the video of step D determined as the detailed surgical step to be referred to in the surgical video of patient 2 is not stored, the cache server (700) may request the video segment management server (800) to transmit the video of step D of the surgical video of patient 2. At this time, based on the surgical schedule of patient 4, the video transmission date and time may be scheduled so that the cache server (700) receives and stores the video of step D of patient 2 before the surgical date and time.

Although various methods and systems according to embodiments of the present invention have been described above as various specific embodiments, these are merely examples. The present invention is not limited to these examples, and should be interpreted as having the broadest scope according to the basic ideas disclosed in this specification. A person of ordinary skill in the art may combine and substitute the embodiments disclosed in the specification to implement patterns of shapes that are not specified, but this also does not deviate from the scope of the present invention. In addition, a person of ordinary skill in the art can easily change or modify the disclosed embodiments based on this specification, and it is obvious that such changes or modifications also fall within the scope of the present invention.

## Claims

1. A method for managing medical images using blockchain, the method comprising:
receiving, at a medical image management server, a medical image recorded in relation to surgery or treatment;
generating, at the medical image management server, a block on a blockchain network based on data related to the medical image; and
updating, through the medical image management server, the block based on new data related to the medical image,
further comprising dividing, at the medical image management server, the medical image into a plurality of segmented images, and sequentially recording the plurality of segmented images in the block based on data related to the plurality of segmented images, wherein the plurality of segmented images are sequentially generated based on timestamp information of each section within the medical image, and
wherein a header of the block comprises: connection link information, which is information related to a connection link accessible to each of the plurality of segmented images; and section information generated based on timestamp information of the start and end points of each of the plurality of segmented images.

2. The method of claim 1, wherein the plurality of segmented images further comprise tag information inserted into the recorded surgical video.

3. The method of claim 2, wherein the tag information comprises at least one of a surgeon, comments related to the surgical situation, a surgical sequence, surgical tools, and surgical events.

4. The method of claim 1, wherein the medical image management server is configured to track accessor information, access IP information, and access time information connected to the connection link as a log record.

5. The method of claim 1, further comprising concluding a transaction contract for the use of the medical image through the medical image management server, wherein the transaction contract is configured to settle costs according to preset transaction conditions using a smart contract.

6. A medical image management server for managing medical images using blockchain, the server comprising:
an image receiving unit configured to receive a medical image recorded in relation to surgery or treatment;
a block generation processing unit configured to generate a block on a blockchain network based on data related to the medical image; and
a block update processing unit configured to update the block based on new data related to the medical image through the medical image management server,
further comprising an image dividing unit configured to divide the medical image into a plurality of segmented images,
wherein the block generation processing unit is configured to sequentially record the plurality of segmented images in the block based on data related to the plurality of segmented images,
wherein the plurality of segmented images are sequentially generated based on timestamp information of each section within the medical image, and
wherein a header of the block comprises: connection link information, which is information related to a connection link accessible to each of the plurality of segmented images; and section information generated based on timestamp information of the start and end points of each of the plurality of segmented images.
